# EUROPÄISCHE PATENTANMELDUNG

(11) **EP 1 985 247 A1**
(43) Veröffentlichungstag der Anmeldung: **29.10.2008**
(21) Anmeldenummer: 08103692.3
(22) Anmeldetag: 23.04.2008
(51) Int. Cl.: A61B 17/34, A61F 2/20, A61M 16/04

(54) **Fisteleinsatz**

(30) Priorität: 27.04.2007 DE 102007021111
(71) Anmelder: TRACOE medical GmbH, 60528 Frankfurt am Main (DE)
(72) Erfinder: Schnell, Ralf, 63500 Seligenstadt (DE)
(74) Vertreter: Seiffert, Klaus

(57) **Zusammenfassung**

Die vorliegende Erfindung betrifft einen Fisteleinsatz (1) mit einem röhrenförmigen Körper (4'), der ein distales (2') und ein proximales Ende (2) und ein zentrales Lumen hat, insbesondere zur Abdichtung bei Fisteln im Zusammenhang mit Stimmprothesen, Sprechventilen, Trachealkanülen, Stoma-Buttons und Konnektoren. Um eine solche Vorrichtung zur Verfügung zu stellen, die beim Einsetzen individuell an die Größe und Form des Stomas bzw. der Fistel anpaßbar ist und eine zuverlässige Abdichtung bewirkt, wird erfindungsgemäß vorgeschlagen, weist der röhrenförmige Körper eine äußere Oberfläche (4) auf, die an die Form einer abzudichtenden Fistel anpaßbar ist, und eine innere Oberfläche (5), die im wesentlichen formstabil ist.

## Beschreibung

Die vorliegende Erfindung betrifft einen Fisteleinsatz mit einem röhrenförmigen Körper, der ein distales und ein proximales Ende und ein zentrales Lumen hat, insbesondere zur Abdichtung bei Fisteln im Zusammenhang mit Stimmprothesen, Sprechventilen, Trachealkanülen, Stoma-Buttons und Konnektoren.

In künstlich angelegte oder auch krankheits- oder fehlbildungsbedingte röhrenförmige Gänge, die Körperöffnungen darstellen oder nur im Körperinneren verlaufen, sogenannte Fisteln oder Stomata, werden häufig Vorrichtungen, wie beispielsweise Ventile, eingesetzt, die eine bestimmte Funktion wahrnehmen sollen. In der Regel wird diese Funktion unterbunden oder beeinträchtigt, wenn die Vorrichtung nicht dicht am Fistelgang anliegt. Insbesondere bei Ventilen besteht im Falle von Leckstellen zwischen Fistel und Ventil die Möglichkeit, daß Flüssigkeiten, Gase oder auch Festkörper an dem Ventil vorbei gelangen könnten und somit dessen Funktion beeinträchtigten.

Ein Beispiel für den Verschluß eines Stomas sind sogenannte Stoma-Buttons. Die Aufgabe dieser Buttons besteht darin, am Tracheostoma eines Patienten möglichst dicht anzuliegen. Überdies verfügen Stoma-Buttons in der Regel über Anschlußmöglichkeiten für Hilfsmittel, wie z.B. Sprechventile oder sogenannte künstliche Nasen (Heat Moisture Exchanger, kurz HME). Diese Hilfsmittel können nur dann effektiv funktionieren, wenn der Stoma-Button für eine ausreichende Abdichtung des Stomas sorgt. Bei unzureichender Abdichtung würden Leckagen eine Stimmbildung bzw. eine effektive Befeuchtung der Atemluft verhindern.

Bekannte Stoma-Buttons gibt es in zahlreichen Längen und Durchmessern. Dennoch ist es nicht möglich, jedes Stoma mit bekannten, handelsüblichen Stoma-Buttons sicher abzudichten, da sich die Stomata von unterschiedlichen Patienten in Größe und Form häufig stark unterscheiden. Eine Fistel oder ein Stoma ist überdies nicht stabil und verändert sich im Lauf der Zeit. Patienten mit übergroßem oder stark deformiertem Stoma fixieren häufig HME bzw. Sprechventile mit Hilfe von selbstklebenden Pflastern mit entsprechenden Adaptern. Der tägliche Wechsel solcher Pflaster führt jedoch häufig zu Hautirritationen am Stoma und ist darüber hinaus auch sehr zeitaufwendig und umständlich.

Auch bei sogenannten Stimmprothesen tritt eine ähnliche Problematik auf. Diese Prothesen werden bei laryngektomierten Patienten, bei denen der Kehlkopf operativ entfernt wurde und somit keine Möglichkeit zu einer natürlichen Stimmbildung mehr besteht, eingesetzt. Bei diesen Patienten wird zwischen Luft- und Speiseröhre eine Fistel angelegt, was ein Hindurchtreten von Luft von der Lunge in den Mundraum ermöglicht. Um zu verhindern, daß Flüssigkeiten oder Speisebrei auf diesem Weg in die Luftröhre und schließlich in die Lunge gelangen können, wird in diese Fistel ein (Einwege-)Ventil, die sogenannte Stimmprothese, eingesetzt.

Bei diesen Stimmprothesen besteht ebenfalls das Problem, daß zwischen Fistelgang und Ventil Leckagen auftreten können. Dies ist für den Patienten nicht nur sehr unangenehm, sondern auch sehr gefährlich, weil dadurch Flüssigkeiten oder Speisebrei an der Stimmprothese vorbei in die Luftröhre und letztlich in die Lunge gelangen können. Eine Möglichkeit der Behebung dieses Problems besteht darin, die Fistel durch eine Naht zusammenzuziehen oder Kollagen in das die Fistel bildende Gewebe einzuspritzen. Insbesondere bei stark deformierten Fisteln sind diese Lösungen jedoch nicht immer erfolgreich. Unter Umständen muß daher die Fistel zur Behebung der Leckage verschlossen werden und an einer anderen Stelle erneut eine Fistel angelegt werden.

Auch eine Verwendung von Stimmprothesen mit sehr großem Durchmesser, die in die Fistel buchstäblich eingeklemmt werden und diese dadurch teilweise aufweiten, ist keine Lösung des Problems, da durch einen zu starken Druck der Prothese auf die Fistel das die Fistel umfassende Gewebe abstirbt und die Fistel dadurch noch stärker erweitert wird.

Es sind zahlreiche unterschiedliche Stimmprothesen bekannt, die insbesondere durch Flansche an den Enden oder Lamellen, die innerhalb der Fistel zu liegen kommen, die Abdichtung verbessern. Jedoch sind auch diese Prothesen nicht direkt beim Einsetzen durch den Arzt oder den Patienten selbst an die Fistel anpaßbar, so daß eine Leckage nicht unmittelbar korrigiert werden kann. Insbesondere unregelmäßig geformte Fisteln lassen sich durch derartige Stimmprothesen, die in der Regel einen kreisrunden Querschnitt aufweisen, nicht ausreichend abdichten.

Der vorliegenden Erfindung liegt daher die Aufgabe zugrunde, eine Vorrichtung zur Abdichtung für Fisteln mit Stimmprothesen, Sprechventilen, Trachealkanülen oder Konnektoren, wie beispielsweise Stoma-Buttons, zur Verfügung zu stellen, die beim Einsetzen individuell an die Größe und Form des Stomas bzw. der Fistel anpaßbar ist und eine zuverlässige Abdichtung bewirkt.

Diese Aufgabe wird durch einen Fisteleinsatz mit einem röhrenförmigen Körper, der ein distales und ein proximales Ende und ein zentrales Lumen hat, wobei der röhrenförmige Körper eine äußere Oberfläche aufweist, die an die Form einer abzudichtenden Fistel anpaßbar ist, und eine innere Oberfläche, die im wesentlichen formstabil ist, gelöst.

Unter innerer Oberfläche ist dabei die Oberfläche zu verstehen, die das zentrale Lumen abgrenzt. Die anpaßbare äußere Oberfläche ist im wesentlichen die Fläche, die mit der Fistel und insbesondere mit dem Fistelgang in Berührung kommt. Selbstverständlich verläuft die Längsachse des zentralen Lumens nach dem Einbringen des Einsatzes in die Fistel im wesentlichen parallel zur Längsausdehnung des Fistelgangs.

Unter formstabil ist hierbei zu verstehen, daß die Form des zentralen Lumens durch einen auf den Einsatz ausgeübten hinreichend großen Druck zwar verformbar ist, jedoch bei einem inneren oder äußeren Überdruck von 100 mbar keine nennenswerte Verformung erfährt.

Das zentrale Lumen des röhrenförmigen Körpers kann der Aufnahme einer Stimmprothese, eines Sprechventils, einer Trachealkanüle oder eines Konnektors dienen. Selbstverständlich kann die formstabile innere Oberfläche aus den Außenwänden der eingesetzten Stimmprothese, des Sprechventils oder des Konnektors bestehen. Das zentrale Lumen kann jedoch auch offen bleiben, sofern der Einsatz lediglich dem Offenhalten der Fistel dient.

Das Lumen des Fisteleinsatzes kann einen kreisrunden Querschnitt aufweisen, denkbar sind jedoch auch ovale, elliptische aber auch eckige oder unregelmäßig geformte Querschnitte. Das Lumen liegt dabei nicht notwendigerweise genau zentral im Querschnitt durch den röhrenförmigen Körper. Insbesondere bei stark verformten Fisteln kann das Lumen aus dem Zentrum verschoben sein, wobei eine möglichst zentrale Lage der inneren Oberfläche und damit des Lumens bevorzugt ist.

Das proximale Ende bezeichnet dabei dasjenige Ende, das bei einem Einsatz für eine Fistel nach dem Einsetzen auf der Seite zu liegen kommt, von der der Einsatz eingebracht wurde. Bei Einsätzen für nach außen ragende Fisteln (äußere Fisteln) ist das proximale Ende daher das aus dem Körper herausragende Ende. Das distale Ende bezeichnet das jeweils gegenüberliegende Ende des röhrenförmigen Körpers.

Bevorzugt beträgt dabei der Abstand der anpaßbaren äußeren Oberfläche von dem proximalen Ende des röhrenförmigen Körpers höchstens 10 mm, bevorzugt höchstens 8 mm, und besonders bevorzugt höchstens 5 mm. Der Fisteleinsatz dient dem Offenhalten von Fisteln bzw. der Anbringung oder dem Einsetzen von etwaigen Hilfsmitteln in eine äußere oder innere Fistel, wobei er so konstruiert ist, daß die anpaßbare äußere Oberfläche in dem Fistelgang zu liegen kommt und diesen bedeckt. Dabei ist es vorteilhaft, wenn die anpaßbare äußere Oberfläche möglichst nahe an dem proximalen Ende ist, so daß dieses nur wenig aus der Fistel herausragt, wodurch das Risiko einer Irritation oder Verletzung der Fistel durch mögliche unkontrollierte Bewegungen verringert wird.

Bei Fisteleinsätzen für innere Fisteln ist es dabei sinnvoll, wenn sowohl das proximale als auch das distale Ende einen geringen Abstand zu der anpaßbaren äußeren Oberfläche haben, der höchstens 10 mm, bevorzugt höchstens 8 mm, und besonders bevorzugt höchstens 5 mm beträgt.

Besonders vorteilhaft ist es aus den oben genannten Gründen, wenn die anpaßbare äußere Oberfläche des röhrenförmigen Körpers einen möglichst ähnlichen Abstand zum distalen und zum proximalen Ende hat, so daß sie auf dem röhrenförmigen Körper weitestgehend zentral zu liegen kommt. Davon umfaßt sind Ausführungsformen, bei welchen die Abstände um etwa 10% zueinander variieren.

Bevorzugt ist auch, daß der röhrenförmige Körper eine Länge von 0,4 cm bis 6 cm, bevorzugt 0,6 cm bis 4 cm aufweist. Für die meisten künstlich angelegten Fisteln ist diese Länge ausreichend, um den Fistelgang vollständig zu durchdringen, so daß auch dadurch erreicht wird, daß der Fisteleinsatz möglichst geringfügig aus dem Fistelgang herausragt.

In einer besonders bevorzugten Ausführungsform des Fisteleinsatzes ist die Länge der anpaßbaren äußeren Oberfläche größer als die Länge des Fistelgangs der Fistel, für die der Einsatz verwendet werden soll. Dadurch bildet die anpaßbare äußere Oberfläche bei der Befüllung und/oder beim Einbringen des Einsatzes in die Fistel an einem oder beiden Enden, außerhalb der abzudichtenden Fistel, einen Flansch aus. Ein solcher Flansch bedeckt den Fistelrand an der jeweiligen Seite vollständig und liegt eng an dem die Fistel umgebenden Gewebe an. Er dient der Fixierung des Fisteleinsatzes in der Fistel. Zusätzlich wird dadurch erschwert, daß an den Rändern der Fistel Flüssigkeiten oder Festkörper, insbesondere Bestandteile von Speisebrei oder Sekret zwischen Fistel und Einsatz eindringen können.

Möglich ist auch, daß unabhängig von der anpaßbaren äußeren Oberfläche an dem distalen und proximalen Ende des Fisteleinsatzes mit der formstabilen inneren Oberfläche verbundene ebenfalls im wesentlichen formstabile Flansche vorhanden sind. Diese formstabilen Flansche können beispielsweise dazu beitragen, daß sich die anpaßbare äußere Oberfläche beim Befüllen nicht in Richtung der zentralen Öffnung ausdehnt und diese verschließt. Dabei dürfen die formstabilen Flansche zumindest soweit sie durch die Fistel hindurchgeführt werden müssen nur eine so geringe radiale Ausdehnung haben, daß beim Einführen des Fisteleinsatzes ein Aufdehnen der Fistel nicht oder nur in geringem Umfang erfolgt. An einem nicht durch die Fistel hindurchzuführenden Ende, dem proximale Ende, kann ein Flansch selbstverständlich auch eine größere Ausdehnung haben.

Endet die Fistel in einem röhrenförmigen Gang, dessen Durchmesser im wesentlichen dem der Fistel entspricht und der eine Verlängerung der Fistel darstellt, wie das beispielsweise bei der Luftröhre, die nach einer Laryngektomie als Öffnung in der Haut endet, der Fall ist, so kann gemäß einer Ausführungsform der Einsatz an dem in diesen Gang hineinreichenden Ende über einen kleineren Flansch verfügen. Dieser kleinere Flansch kann auch lediglich aus dem oben beschriebenen formstabilen Flansch bestehen, der unabhängig von der anpaßbaren äußeren Oberfläche ist.

Zweckmäßig ist es, wenn in der anpaßbaren äußeren Oberfläche der Flansch oder die Flansche im unbefüllten Zustand des Fisteleinsatzes und/oder vor dem Einbringen des Einsatzes in die Fistel vorgeformt sind. Dabei ist es sinnvoll, wenn auch die vorgeformten Flansche anpaßbar sind, so daß sich diese nach dem Einbringen des Einsatzes in die Fistel, bzw. nach dem Befüllen des Einsatzes in der Fistel eng an die Fistelränder anlegen und diese abdichten.

Denkbar ist dabei auch, daß die Flansche zusammen mit oder getrennt von dem röhrenförmigen Körper befüllbar sind und dadurch an die Fistelränder anpaßbar sind.

In einer bevorzugten Ausführungsform besteht die äußere Oberfläche aus einem geschlossenporigen Schaumstoff. Es besteht auch die Möglichkeit, daß sowohl innere als auch äußere Oberfläche aus einem geschlossenporigen Schaumstoff bestehen oder aber auch die gesamte Oberfläche des röhrenförmigen Körpers.

Unter Oberflächen aus geschlossenporigem Schaumstoff ist dabei ein geschlossenzelliger Schaumstoff zu verstehen, der lediglich die Oberfläche des Fisteleinsatzes bedeckt.

Der Fisteleinsatz kann jedoch auch aus einem Integralschaum bestehen, bei welchem eine geschlossenzellige Außenhaut einen offenzelligen Kern einfaßt. Es sind auch Mischungen aus unterschiedlichen Schaumstofftypen denkbar. Die äußere Oberfläche sollte jedoch in jedem Fall aus einem verformbaren Weichschaum bestehen, so daß diese Oberfläche an die Form der Fistel anpaßbar ist.

Durch den Schaumstoff ist insbesondere eine gute Anpaßbarkeit des Einsatzes an die Form einer abzudichtenden Fistel gegeben. Die geschlossenporige äußere Oberfläche verhindert dabei das Eindringen von Flüssigkeit oder auch Festkörpern, insbesondere Speisebrei, in den Einsatz und liegt gleichzeitig so eng an der Fistel an, daß auch zwischen Fistel und Einsatz ein Durchdringen von Flüssigkeiten oder Festkörpern nicht möglich ist. Auch das Durchströmen von Gas durch den Einsatz oder zwischen Einsatz und Fistel kann durch einen solchen Schaumstoff verhindert werden. Der Schaumstoff soll dabei jedoch so weich sein, daß der auf das Gewebe ausgeübte Druck weniger als 30 mbar beträgt.

Bei einer weiteren bevorzugten Ausführungsform besteht zumindest die äußere Oberfläche des Fisteleinsatzes aus einer Außenhaut, die zusammen mit der das zentrale Lumen abgrenzenden inneren Oberfläche ein Volumen abgrenzt, welches befüllbar ist und wobei die äußere Oberfläche durch Befüllung des Volumens in ihrer Form an eine abzudichtende Fistel anpaßbar ist.

Der Vorteil dieser Ausführungsform liegt darin, daß das Volumen bei der Befüllung so einstellbar ist, daß die anpaßbare äußere Oberfläche genau und am gesamten Umfang mit dem Fistelgang in Berührung kommt und so die Fistel abdichtet.

Dabei kann die Außenhaut insbesondere aus einer dünnen Folie bestehen.

Diese Folie sollte in einer Ausführungsform aus einem flexiblen und/oder elastischen Material bestehen.

Im übrigen kann dadurch als Material der äußeren Oberfläche ein Material verwendet werden, das im wesentlichen dazu dient, das Eindringen von Flüssigkeiten, Festkörpern oder Gasen in den Einsatz zu verhindern, und dazu in der Lage ist, sich eng an die Fistel anzulegen. Das Material, mit dem der Einsatz befüllt wird, kann demgegenüber im wesentlichen Eigenschaften aufweisen, die einen besonders gleichmäßigen Druck des Einsatzes auf die Fistel ermöglichen und durch die das eigentliche Abdichten zwischen Fistel und Einsatz bewirkt wird.

Bei befüllbaren Fisteleinsätzen kann eine Einstellung des Druckes auf die Fistelwand über die Befüllmenge erfolgen. Der Druck, den der Einsatz auf die Fistelwand ausübt, soll ausreichend sein, um den Anforderungen des Einsatzes standzuhalten. Dabei ist abzuwägen zwischen einem ausreichenden hohen Druck, um die Dichtigkeit des Fisteleinsatzes zu gewähren und insbesondere ein Aspirieren des Einsatzes durch den Patienten zu verhindern, und einem hinreichend geringen Druck, der eine Schädigung des die Fistel umgebenden Gewebes verhindert. Der Intakterhaltung des Gewebes ist im Zweifel dabei der Vorzug zu geben.

Die Außenhaut kann sich dabei lediglich über die anpaßbare äußere Oberfläche oder auch über weitere Teile oder die gesamte Oberfläche des Einsatzes erstrecken, sofern durch die Außenhaut und weitere Bestandteile des Einsatzes, oder der Sprechventile, Stimmprothesen oder Konnektoren ein Volumen geschaffen wird, welches befüllbar ist.

Besonders bevorzugt besteht die Außenhaut aus einer dünnen Folie, die in der Lage ist, das Volumen abzugrenzen, ohne selbst durch ihre Dicke wesentlich zu dem Volumen beizutragen. Selbstverständlich ist diese Folie haut- bzw. körperverträglich. Die Folie dient dabei im wesentlichen der Übertragung der Eigenschaften, insbesondere des Druckes, des Füllmaterials auf die Fistelwand. Überdies ist sie in der Lage, den Einsatz selbst gut gegenüber der Umgebung abzudichten. Die Dicke der Folie soll dazu 5 µm nicht unter- und 2 mm nicht überschreiten und bevorzugt zwischen 75 und 900 µm betragen.

Dabei ist es besonders vorteilhaft, wenn die Folie aus einem flexiblen und/oder elastischen Material besteht. Diese Materialien sind besonders gut in der Lage, sich an die Form einer Fistel anzupassen. Die Möglichkeit einer elastischen Dehnung der Folie bei einem elastischen Material kann insbesondere bei sehr großen Fisteln bei der Abdichtung hilfreich sein. Dabei soll der Einsatz so befüllt sein, daß auf die Fistel, insbesondere den Fistelgang, die Fistelränder und das umgebende Gewebe nur ein moderater Druck ausgeübt wird, der keine Nekrose des Gewebes zur Folge hat. Die Folie muß sich dabei an die Kontur der Fistel anpassen und sich bereits bei geringen Drücken von weniger als 100 mbar, bevorzugt von weniger als 50 mbar, besonders bevorzugt von zwischen 15 und 30 mbar, dehnen. Der sich daraus ergebende geringfügige Widerstand der Folie gegenüber einer Dehnung ermöglicht eine genauere Bestimmung des auf das die Fistel umgebende Gewebe ausgeübten Druckes, da der Dehnungswiderstand der Folie dem durch die Befüllung ausgeübten Druck entgegenwirkt.

Bei der Verwendung einer elastisch dehnbaren Folie sind unterschiedliche Ausgestaltungen des Fisteleinsatzes denkbar. Der Fisteleinsatz könnte beispielsweise vor dem Einsetzen bereits befüllt sein. Dabei ist sein äußerer Durchmesser vor dem Einsetzen größer als der Durchmesser des Fistelganges. Beim Einsetzen wird der Fisteleinsatz abschnittsweise zusammengedrückt und die elastische dehnbare Folie zieht sich in diesen Bereichen zusammen und liegt dadurch faltenfrei am Fistelgang an.

Für das faltenfreie Anliegen der elastisch dehnbaren Folie am Fistelgang ist es nötig, daß der Fisteleinsatz bei einer Befüllung, bis die Außenhaut faltenfrei aber noch nicht gedehnt ist einen äußeren Durchmesser aufweist, der kleiner ist als der Durchmesser der Fistel oder diesem entspricht.

Denkbar als Folienmaterial wären für bestimmte Ausführungsformen auch Folien, die nicht dehnbar oder in bestimmten Druckbereichen plastisch dehnbar sind.

Die Folien können dabei aus vielen unterschiedlichen Materialien oder auch Materialmischungen bestehen. Beispiele geeigneter Materialien sind Polyurethan, Polypropylen, Polyethylen, Polyethylenterephthalat, Polyvinylchlorid oder andere Polymere und Mischungen aus Polymeren (Blends) sowie SEBS (Styrol-Ethen-Buten-Styrol), SBS (Styrol-Butadien-Styrol), SIS (Styrol-Isopren-Styrol), IR (Polyisopren) oder andere thermoplastische Elastomere, Latex, Silicon, Naturgummi oder synthetischer Gummi.

Bevorzugt ist auch, daß das durch die Außenhaut abgegrenzte Volumen mit einem Fluid oder Gas befüllbar ist. Sowohl Flüssigkeiten, als auch Gase verteilen den Druck des Einsatzes auf die Fistel durch die Außenhaut gleichmäßig, so daß eine besonders gute Abdichtung erfolgen kann. Der Einsatz könnte aber auch mit einem Gel befüllt werden, das die gleichen Eigenschaften aufweist.

Besonders bevorzugt ist dabei, wenn das Volumen mit einer aushärtenden Flüssigkeit befüllt werden kann. Derartige Flüssigkeiten können beispielsweise Harze oder Kautschuke sein, die durch eine chemische Reaktion innerhalb des Einsatzes polymerisieren. Dazu kommen sowohl Ein- als auch Mehrkomponentensysteme beispielsweise aus Polyurethan oder Silikon in Frage.

Denkbar sind aber auch Materialien, die einen Erstarrungspunkt bei etwa 42 °C bis 55 °C haben, so daß sie in flüssigem Zustand eine Temperatur aufweisen, die für den Patienten grundsätzlich noch erträglich ist, und die nicht zu einer Zerstörung des Gewebes führt. Ein Beispiel hierfür wären bestimmte Arten von Paraffin, Stearin, Bienenwachs oder auch Polyethylenglykol, z.B. PEG 1000, PEG 1500, PEG 2000 oder PEG 4000 oder Mischungen daraus. Bei Körpertemperatur erstarren diese Materialien bzw. sind diese Materialien in ihrem festen Aggregatszustand.

Auch ein Kristallisationsvorgang innerhalb des Einsatzes wäre denkbar. Dabei wird das Volumen mit einer übersättigten Schmelze befüllt. Durch Erzeugung oder Freisetzung von Kristallisationskeimen erfolgt eine gezielte Erstarrung des Materials aufgrund des einsetzenden Kristallisationsvorgangs. Ein mögliches Material mit derartigen Eigenschaften für die Befüllung wäre beispielsweise Natriumacetattrihydrat.

Ein wesentlicher Vorteil der Verwendung von Materialien, die erstarren oder kristallisieren, ist, daß sich die Verformbarkeit des Materials durch erneutes Erwärmen wieder herstellen läßt, so daß eine nochmalige Anpassung durch Erwärmen und Abkühlen möglich ist.

Die erstarrten Materialien sind dabei bevorzugt in geringem Maße elastisch oder plastisch verformbar, so daß der Einsatz beispielsweise zur Reinigung unter Zusammendrücken entnommen werden kann, ohne das die Fistel umrandende Gewebe stark zu dehnen. Andererseits sollte die Flexibilität aber auch so gering sein, daß der Einsatz grundsätzlich dicht an der Fistel anliegt.

In einer weiteren bevorzugten Ausführungsform ist der Einsatz mit einem Schaumstoff befüllt. Zweckmäßig handelt es sich dabei um einen Weichschaumstoff, der durch geringen Druck verformbar ist. Ein Eindringen von Flüssigkeiten oder Speisebrei in den Schaumstoff wird bei dieser Ausführungsform durch die Außenhaut verhindert. Der Schaumstoff kann dabei beispielsweise durch chemische Reaktion nach dem Befüllen des Einsatzes, wenn sich dieser bereits in der Fistel befindet, gebildet werden. Bei einem derartig weichen Material ist es jedoch auch denkbar, daß der Schaumstoff bereits bei der Herstellung des Einsatzes in das von der Außenhaut abgegrenzte Volumen eingebracht wird und beim Einbringen des Einsatzes in die Fistel eine Abdichtung durch die leichte elastische Verformbarkeit des Schaumstoffs erfolgt. Durch die Vielzahl an Zellen oder Kammern, aus denen ein solcher Schaumstoff besteht, die unabhängig voneinander komprimiert werden können, kann sich ein solcher Schaumstoff gut an unregelmäßig geformte Fisteln anpassen.

Bei mit Weichschaumstoff befüllten Einsätzen, die durch eine Folie abgegrenzt sind, besteht auch die Möglichkeit, die in dem Schaumstoff befindliche Luft vor dem Einsetzen in die Fistel abzusaugen, wodurch sich das Volumen verkleinert und dadurch das Einsetzen erleichtert wird. Nach der Positionierung des Fisteleinsatzes in der Fistel kann ein Einströmen der Luft wieder ermöglicht werden, wobei sich der Einsatz quasi selbst befüllt und dadurch eng an die Fistel und insbesondere den Fistelgang angedrückt wird.

Besonders bevorzugt erfolgt ein Verbinden der Stimmprothesen, Sprechventile oder Konnektoren mit dem Einsatz durch Verkleben oder Verschweißen. Dabei handelt es sich um hinreichend stabile Arten der Verbindung, die gleichzeitig eine Abdichtung zwischen dem Einsatz und der jeweiligen im zentralen Lumen enthaltenen Vorrichtung bilden. Denkbar wäre jedoch auch, daß die Stimmprothesen, Sprechventile oder Konnektoren in das zentrale Lumen mit Hilfe eines Gewindes eingeschraubt werden. Zweckmäßig ist es auch, wenn das zentrale Lumen als Rohr mit Konnektor ausgestaltet ist, auf welchen Stimmprothesen oder Sprechventile aufgesetzt werden können.

In einer weiteren bevorzugten Ausführungsform der Erfindung ist die äußere Oberfläche des Einsatzes mit einem Gleitmittel versehen. Dadurch wird insbesondere das Einführen des Einsatzes in die Fistel erleichtert. Um ein leichtes Herausgleiten des Einsatzes zu verhindern kann dabei ein wasserlösliches Gleitmittel verwendet werden, das innerhalb des Körpers nur eine kurze Zeit auf der äußeren Oberfläche des Einsatzes verbleibt. Ein solches Gleitmittel kann aber auch lediglich zur Einführung des Einsatzes auf die Oberfläche aufgetragen werden.

Zweckmäßig ist es weiterhin, wenn der Druck, den die Abdichtung auf die Fistelwand ausübt, einstellbar ist, so daß ein zu starker Druck auf das an die Fistel angrenzende Gewebe und eine dadurch möglicherweise entstehende Nekrose verhindert wird. Eine solche Einstellbarkeit ist bei den im wesentlichen aus Schaumstoff bestehenden Einsätzen durch die Art des Schaumstoffs und die Größe des Durchmessers des Einsatzes im Vergleich zu dem Durchmesser der Fistel, für die der Einsatz verwendet werden soll, möglich, wobei der Druck auf die Fistelwand etwa 10 bis 25 mbar betragen soll.

Besonders bevorzugt ist dabei der Außendurchmesser des Bereichs des röhrenförmigen Körpers, der innerhalb der Fistel zu liegen kommt und am Fistelgang anliegt, ohne äußere Einschränkung befüllt größer als der Durchmesser der Fistel. Insbesondere durch eine solche Ausgestaltung des Einsatzes ist es möglich, eine gute Abdichtung bei gleichzeitig sehr geringem Druck des Einsatzes auf den Fistelgang und damit das umliegende Gewebe zu erreichen.

Die äußere Oberfläche des Einsatzes kann dabei in kleinen Falten an dem Fistelgang anliegen, die jedoch durch die Wahl eines geeigneten Materials der Oberfläche eine so geringe Ausdehnung aufweisen, daß ein Durchtreten von Flüssigkeiten oder Gasen durch die Falten unterbunden wird. Insbesondere bei der Verwendung von dünnen Folien als Material für eine Außenhaut soll dabei die Dicke der Folie so gewählt werden, daß diese im Bereich zwischen 5 µm und 150 µm, bevorzugt zwischen 10 und 70 µm, liegt, wodurch eine hinreichende mechanische Stabilität bei gleichzeitig ausreichender Flexibilität gewährleistet ist. Die Folie ist dabei im wesentlichen nicht dehnbar, so daß der Druck, der auf das die Fistel umgebende Gewebe ausgeübt wird unmittelbar durch Messung des Innendrucks bei der Befüllung feststellbar ist.

Die Stabilität dieser Ausführungsform kann durch das Befüllen mit einer aushärtenden Flüssigkeit verbessert werden.

Weitere Vorteile, Merkmale und Anwendungsmöglichkeiten der vorliegenden Erfindung werden deutlich anhand der folgenden Beschreibung bevorzugter Ausführungsformen und den dazugehörigen Figuren.

Es zeigen:

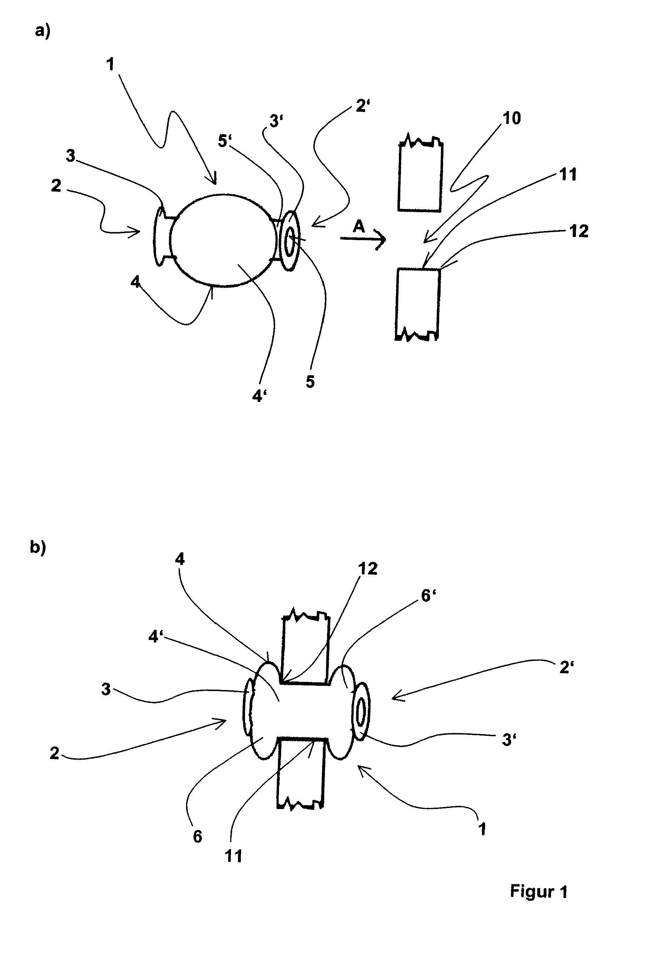

- Figur 1a:: Eine schematische Darstellung eines Längsschnitts durch eine Fistel und eines erfindungsgemäßen Fisteleinsatzes vor dem Einsetzen,
- Figur 1 b:: Eine schematische Darstellung eines Längsschnitts durch eine Fistel und des erfindungsgemäßen Fisteleinsatzes entsprechend Figur 1a nach dem Einsetzen,
- Figur 2a:: Eine schematische Darstellung eines Längsschnitts durch eine Fistel und einer andere Ausführungsform eines erfindungsgemäßen Fisteleinsatzes vor dem Einsetzen,
- Figur 2b:: Eine schematische Darstellung eines Längsschnitts durch eine Fistel und des erfindungsgemäßen Fisteleinsatzes entsprechend Figur 2a nach dem Positionieren in der Fistel und
- Figur 2c:: Eine schematische Darstellung eines Längschnitts durch eine Fistel und des erfindungsgemäßen Fisteleinsatzes entsprechend Figur 2a nach dem Befüllen.

In Figur 1 a ist ein erfindungsgemäßer Fisteleinsatz 1 mit einem proximalen Ende 2 und einem distalen Ende 2' schematisch in Aufsicht dargestellt. Er verfügt über eine äußere Oberfläche 4, welche an die Form der abzudichtenden Fistel 10 anpaßbar ist.

Bei er in Figur 1 dargestellten Ausführungsform der Erfindung wird die äußere Oberfläche 4, welche an die Form der abzudichtenden Fistel 10 anpaßbar ist, aus einem Körper 4' aus Schaumstoff gebildet. Es handelt sich dabei im wesentlichen um einen offenporigen Schaumstoff, der von einer gas- und flüssigkeitsundurchlässigen Außenhaut begrenzt wird, die die eigentliche anpaßbare Oberfläche bildet. Bei der Außenhaut kann es sich beispielsweise um einen geschlossenporigen Schaumstoff handeln. Es könnte jedoch auch eine dünne, flexible, elastische Folie sein, die aus PU, Polypropylen, PE, Polyethylenterephthalat, PVC, SEBS, SBS, SIS, IR, natürlichem oder künstlichen Kautschuk, Latex, Silicon oder Mischungen daraus oder Materialien mit ähnlichen Eigenschaften, die körperverträglich sind, bestehen kann.

Der die anpaßbare äußere Oberfläche bildende Körper 4' erstreckt sich um eine im wesentlichen formstabile innere Oberfläche 5 herum, die von einem ebenfalls röhrenförmigen Körper 5' gebildet wird. Der röhrenförmige Körper 5', der die innere Oberfläche bildet, ragt an beiden Enden 2 und 2' über den die äußere Oberfläche 4 bildenden Körper 4' hinaus. An den beiden Enden 2 und 2' sind jeweils Flansche 3 und 3' vorgeformt, deren äußerer Durchmesser jedoch nicht größer ist als der Innendurchmesser der Fistel 10, der durch die Fistelränder 12 und den Fistelgang 11 festgelegt ist.

Der Pfeil A gibt die Richtung an, in welcher der Fisteleinsatz 1 in die Fistel 10 verbracht wird.

Figur 1b stellt den in die Fistel 10 eingebrachten Fisteleinsatz 1 aus Figur 1a in einer Fistel dar. Der Einfachheit halber ist die Fistel im Längsschnitt dargestellt. Der Einsatz ist jedoch wie in Figur 1 a schematisch in einer seitlichen Aufsicht zu sehen.

Durch die Verformbarkeit des Körpers 4' mit der äußeren Oberfläche 4 kann der Fisteleinsatz 1 ohne Aufweitung des Fistelrandes 12 oder des Fistelganges 11 in die Fistel 10 eingebracht werden. Dabei haben sich durch die Verdrängung des Materials des Körpers 4', welches sich unter der äußeren Oberfläche 4 befindet, an beiden Seiten der Fistel Flansche 6 und 6' ausgebildet. Die vorgeformten Flansche 3 und 3' an dem Körper 5', die wie die innere Oberfläche 5 im wesentlichen formstabil sind, verhindern dabei, daß das Material des Körpers 4' unter der äußeren Oberfläche 4 zu sehr in Richtung eines der Enden 2 oder 2' verschoben wird. Dadurch wird ein vollständiges oder teilweises Verschließen des zentralen Lumens des röhrenförmigen Körpers des Fisteleinsatzes 1 durch die anpaßbare Oberfläche 4 verhindert.

Die Flansche 6 und 6' liegen auf beiden Seiten der Fistel 10 eng an den Fistelrändern 12 an. Gleichzeitig hat sich die Oberfläche 4 dem Fistelgang 11 angepaßt, wobei das Material des Körper 4' teilweise komprimiert wird und dadurch mit Druck an dem Fistelgang 11 anliegt. Durch die Auswahl eines geeigneten Materials sowie eines geeigneten Durchmessers des Körpers 4' wenn er sich ohne Begrenzung auffalten kann, beträgt der Anpreßdruck der äußeren Oberfläche 4 auf den Fistelgang 11 etwa 10 bis 25 mbar, so daß eine ausreichende Abdichtung erzielt wird und gleichzeitig der Druck, der auf das Gewebe ausgeübt wird, nicht so groß ist, daß er zu Gewebeschäden führen würde. Bei derartigen Fisteleinsätzen 1 ist eine zusätzliche Kontrolle des Druckes überflüssig.

Bei weiteren möglichen Ausführungsform, die den in den Figuren 1a und 1b dargestellten Fisteleinsätzen entsprechen, ist der Körper 4' mit einem Material gefüllt, welches in der Lage ist, auszuhärten. Dabei entsteht ein im wesentlichen formstabiler Körper, dessen Oberfläche nach dem Aushärten nicht mehr ohne weiteres anpaßbar ist. Als Füllmaterialien kommen z.B. Materialien in Frage, die reversibel aushärten, wie beispielsweise ein Polyethylenglykol (PEG) welches eine Erstarrungstemperatur von über 40°C hat oder eine übersättigte Schmelze, oder die durch eine Polymerisationsreaktion irreversibel aushärten, wobei das Material so gewählt wird, daß zumindest eine geringfügige elastische oder plastische Verformbarkeit noch möglich ist. Derartige polymerisierende Materialien sind beispielsweise ein- oder mehrkomponentige Silikonkautschuke oder Polyurethanharze.

Entsprechend der oben beschriebenen, mit Schaumstoff gefüllten Ausführungsform, besteht die Außenhaut des Körpers 4', die die äußere Oberfläche 4 bildet aus einer dünnen, flexiblen, elastisch dehnbaren Folie. Diese Folie wird beim Einsetzen des Fisteleinsatzes abschnittsweise gedehnt und liegt daher ohne Falten an den Fistelrändern und dem Fistelgang an.

Die Fisteleinsätze 1 mit aushärtenden Körpern 4' werden in verformbarem Zustand in die Fistel 10 eingesetzt. Die Aushärtung erfolgt, wenn sich der Einsatz wie in Figur 1b dargestellt bereits in der Fistel befindet. Dabei wird das Füllmaterial so gewählt, daß die Aushärtung mit einer gewissen Verzögerung erfolgt, so daß eventuelle nötige Korrekturen in Bezug auf den Sitz des Einsatzes 1 in der Fistel 10 oder die Verteilung des Materials auf die Flansche 6 und 6' noch möglich sind.

Fisteleinsätze mit reversibel aushärtenden Körpern 4' können durch Wiederverflüssigung oder Wiederherstellung der Verformbarkeit des Materials leicht aus der Fistel 10 entnommen werden. Bei irreversibel ausgehärteten Fisteleinsätzen wird die Entnahme des Einsatzes durch die geringfügig vorhandene elastische oder plastische Verformbarkeit ermöglicht.

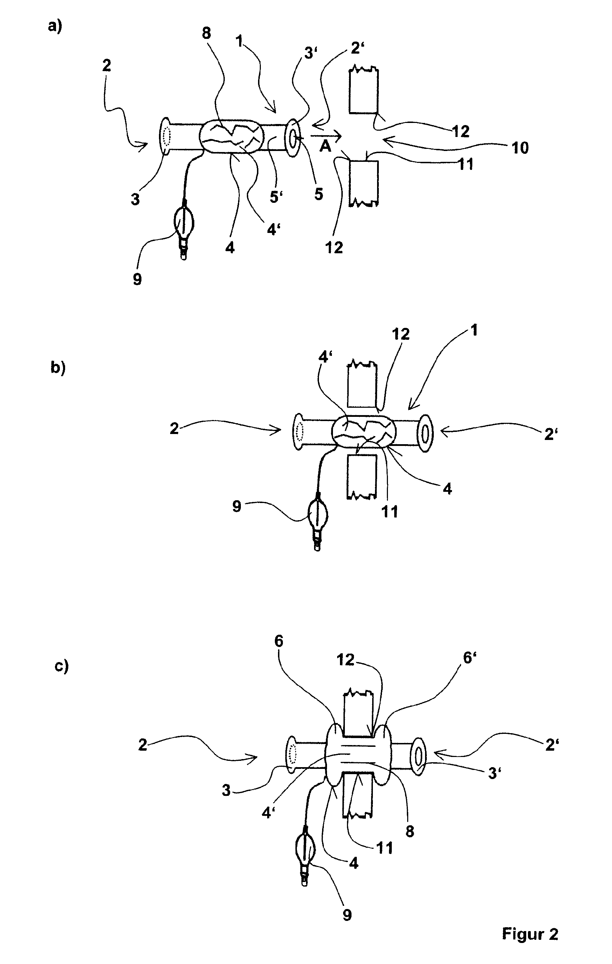

In Figur 2a ist ein Fisteleinsatz 1 dargestellt, welcher über einen röhrenförmigen Körper verfügt, mit einer inneren Oberfläche 5 und einer äußeren Oberfläche 4, die an die Kontur einer Fistel 10 anpaßbar ist. Die innere Oberfläche 5 wird dabei von einem röhrenförmigen Körper 5' gebildet, der ein proximales Ende 2 und ein distales Ende 2' aufweist und von einem röhrenförmigen Körper 4' partiell umhüllt ist, welcher die anpaßbare Oberfläche 4 bildet. Der röhrenförmige Körper 4' befindet sich im wesentlichen zentral auf dem Umfang des röhrenförmigen Körpers 5', ist befüllbar und liegt in dem in Figur 2a dargestellten unbefüllten Zustand in Falten 8. Zur Befüllung des Körpers 4' ist ein Ventil 9 vorgesehen. Bei einer Befüllung des Körpers 4' in frei entfaltbarem Zustand, wie in Figur 1a dargestellt, würde der Außendurchmesser des Körpers 4' größer sein als der Innendurchmesser der Fistel 10. Der Körper 5' weist an den Enden flanschartige Verdickungen 3 und 3' auf, die der Stabilisierung dienen und für die Befestigung von Sprechventilen und/oder Feuchte-/Wärmeaustauschern vorgesehen sind.

Der Fisteleinsatz 1 kann in Richtung des Pfeils A in die Fistel 10 eingebracht werden.

In Figur 2b ist der Fisteleinsatz 1 aus Figur 2a dargestellt, wie er in die Fistel hineinverschoben ist. Der Außendurchmesser des Fisteleinsatzes 1 einschließlich der äußeren Oberfläche 4 ist dabei sowohl im Bereich der Fistelränder 12 als auch des Fistelganges 11 kleiner als der Innendurchmesser der Fistel 10. Der röhrenförmige Körper 4' befindet sich noch in unbefülltem Zustand und weist einen unregelmäßigen Faltenwurf 8 auf. Die Außenhaut, des Körpers 4', die die anpaßbare Oberfläche 4 darstellt, besteht aus einer sehr flexiblen, weichen Folie, die auch geringfügig dehnbar sein kann. Sie ist körperverträglich und gas- sowie flüssigkeitsundurchlässig.

Die Befüllung des Körpers 4' kann durch das Ventil 9 erfolgen. Dabei können verschiedene Gase, u. a. Luft, oder ein Fluid eingefüllt werden. Der Vorteil einer solchen Befüllung besteht darin, daß für eine Entnahme des Fisteleinsatzes 1 aus der Fistel 10 lediglich zumindest ein Teil des Gases oder des Fluids durch das Ventil 9 entnommen werden muß. Möglich ist jedoch auch, daß ein aushärtendes Material eingefüllt wird. Hierfür sind beispielsweise die oben beschriebenen aushärtenden Materialien geeignet. Derartige mit einem aushärtenden Material befüllten Fisteleinsätze sind im Gegensatz zu den mit einem Gas oder einem Fluid befüllten Einsätzen, bei denen das Ventil 9 an dem Körper 4' verbleiben sollte, auch für innere Fisteln geeignet, da das innerhalb des Körpers störende Ventil 9 nach dem Aushärten des Materials von dem Körper 4' entfernt werden kann.

Figur 2c stellt den befüllten Fisteleinsatz 1 dar, der dem Einsatz der Figuren 2a und 2b entspricht. Da die Länge des Körpers 4' und damit der anpaßbaren Oberfläche 4 größer ist, als die Länge des Fistelganges 11, haben sich durch die Befüllung des Körper 4' an beiden Enden Flansche 6 und 6' ausgebildet. Dadurch daß der Körper 4 bei Befüllung ohne äußere Begrenzung einen größeren Außendurchmesser hat als der Innendurchmesser der Fistel 10, für die der Fisteleinsatz verwendet werden soll, liegt die anpaßbare Oberfläche 4 in befülltem Zustand des Körpers 4' in Falten an dem Fistelgang und dem Fistelrand an. Durch die große Flexibilität der Folie sind diese Falten jedoch so klein und weisen einen so geringen Durchmesser am Faltengrund auf, daß eine Abdichtung der Fistel um den Fisteleinsatz herum gegenüber Gas und Flüssigkeit gegeben ist.

Die sich bei der Befüllung ausbildenden Flansche 6 und 6' liegen eng an dem Fistelrand und dem die Fistel umgebenden Gewebe an. Der Abstand zwischen diesen durch die Befüllung ausgebildeten Flansche von den an den Enden 2 und 2' vorhandenen Verdickungen 3 und 3' beträgt bei dieser Ausführungsform weniger als 1 cm, häufig weniger als 0,5 cm. Dadurch wird die Verletzungsgefahr durch die überstehenden Enden des Körpers 5', der die innere Oberfläche 5 bildet minimiert, was insbesondere bei Einsätzen für innere Fisteln wichtig ist. Auch bei äußeren Fisteln sollte insbesondere der Abstand an dem proximalen Ende des Einsatzes die oben genannten Werte nicht überschreiten, so daß der Einsatz nicht unnötig weit aus der Körperöffnung herausragt.

Der Körper 5', der die innere Oberfläche bildet kann bei den Ausführungsformen in den Figuren 1 und 2 aus einem Stoma-Button oder einer Stimmprothese bestehen. Der Körper 4' ist damit durch Verschweißen oder Verkleben verbunden.

Weitere mögliche Ausführungsformen sind denkbar, bei denen eine Stimmprothese an der inneren Oberfläche 5 durch Verschweißen oder Verkleben innerhalb des röhrenförmigen Körpers 5' angebracht ist.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Fisteleinsatz (1) mit einem röhrenförmigen Körper, der ein distales (2') und ein proximales Ende (2) und ein zentrales Lumen hat, wobei der röhrenförmige Körper eine äußere Oberfläche (4) aufweist, die an die Form einer abzudichtenden Fistel (10) anpaßbar ist, und eine innere Oberfläche (5), die im wesentlichen formstabil ist, insbesondere zur Abdichtung bei Fisteln im Zusammenhang mit Stimmprothesen, Sprechventilen, Trachealkanülen, Stoma-Buttons und Konnektoren.

2. Fisteleinsatz (1) nach Anspruch 1, **dadurch gekennzeichnet, daß** der Abstand zwischen dem proximalen Ende (2) des röhrenförmigen Körpers und der anpaßbaren äußeren Oberfläche (4) des röhrenförmigen Körpers höchstens 10 mm, bevorzugt höchstens 8 mm, besonders bevorzugt höchstens 5 mm beträgt.

3. Fisteleinsatz (1) nach Anspruch 1 oder 2, **dadurch gekennzeichnet, daß** die anpaßbare äußere Oberfläche (4) des röhrenförmigen Körpers im wesentlichen zentral zwischen den beiden Enden (2, 2') angeordnet ist.

4. Fisteleinsatz (1) nach einem der vorangehenden Ansprüche, **dadurch gekennzeichnet, daß** der röhrenförmige Körper eine Länge von 0,4 cm bis 6 cm, bevorzugt von 0,5 cm bis 4 cm aufweist.

5. Fisteleinsatz (1) nach einem der vorangehenden Ansprüche, **dadurch gekennzeichnet, daß** die Länge der anpaßbaren äußeren Oberfläche (4) größer ist als die Länge des Fistelgangs (11) der Fistel (10) für die der Einsatz (1) verwendet werden soll.

6. Fisteleinsatz (1) nach einem der vorangehenden Ansprüche, **dadurch gekennzeichnet, daß** in der anpaßbaren äußeren Oberfläche (4) vor dem Einbringen des Einsatzes (1) in die Fistel (10) an einem oder beiden Enden ein Flansch (6, 6') vorgeformt ist.

7. Fisteleinsatz (1) nach einem der vorangehenden Ansprüche, **dadurch gekennzeichnet, daß** zumindest die anpaßbare äußere Oberfläche (4) von einem geschlossenporigen Schaumstoff begrenzt wird.

8. Fisteleinsatz (1) nach einem der Ansprüche 1 bis 6, **dadurch gekennzeichnet, daß** die anpaßbare äußere Oberfläche (4) von einer Außenhaut gebildet wird, die ein Volumen abgrenzt, welches befüllbar ist und durch Befüllung in ihrer äußeren Form an eine abzudichtende Fistel (10) anpaßbar ist.

9. Fisteleinsatz (1) nach einem der vorangehenden Ansprüche, **dadurch gekennzeichnet, daß** der Einsatz mit einem Fluid oder Gas befüllbar ist.

10. Fisteleinsatz (1) nach Anspruch 9, **dadurch gekennzeichnet, daß** das Fluid eine aushärtende Flüssigkeit ist.

11. Fisteleinsatz (1) nach einem der Ansprüche 1 bis 6 oder 8, **dadurch gekennzeichnet, daß** der Einsatz mit einem Schaumstoff befüllt ist.

12. Fisteleinsatz (1) nach einem der vorangehenden Ansprüche, **dadurch gekennzeichnet, daß** eine Verbindung mit den Stimmprothesen, Sprechventilen, Trachealkanülen und Konnektoren durch Verkleben oder Verschweißen erfolgt.

13. Fisteleinsatz (1) nach einem der vorangehenden Ansprüche, **dadurch gekennzeichnet, daß** die äußere Oberfläche mit einem Gleitmittel versehen ist.

14. Fisteleinsatz (1) nach einem der vorangehenden Ansprüche, **dadurch gekennzeichnet, daß** der Druck, den die äußere Oberfläche (4) auf die Fistelwand (11) ausübt, einstellbar ist.

15. Fisteleinsatz (1) nach einem der vorangehenden Ansprüche, **dadurch gekennzeichnet, daß** der Außendurchmesser des Bereichs des röhrenförmigen Körpers, der innerhalb der Fistel zu liegen kommt und am Fistelgang anliegt, ohne äußere Einschränkung befüllt größer ist als der Durchmesser der Fistel für die der Einsatz verwendet werden soll.
